# EUROPÄISCHE PATENTSCHRIFT

(11) **EP 0 649 295 B1**
(45) Veröffentlichungstag und Bekanntmachung des Hinweises auf die Patenterteilung: **18.03.1998**
(21) Anmeldenummer: 93915858.0
(22) Anmeldetag: 09.07.1993
(51) Int. Cl.: A61F 2/24

(54) **STÜTZGEHÄUSE FÜR KLAPPEN- UND SCHLIESSORGANE, INSBESONDERE, FÜR HERZKLAPPENPROTHESEN**
SUPPORT HOUSING FOR VALVE AND CLOSURE MEMBERS
BOITIER DE SUPPORT D'ORGANES OBTURATEURS ET VALVULAIRES

(30) Priorität: 10.07.1992 DE 4222610
(43) Veröffentlichungstag der Anmeldung: 26.04.1995
(73) Patentinhaber: Adiam Medizintechnik GmbH & Co. KG, D-41812 Erkelenz (DE)
(72) Erfinder: Jansen, Josef, D-52064 Aachen (DE); Jansen, Ulrich, D-40225 Düsseldorf (DE)
(74) Vertreter: Vomberg, Friedhelm, Dipl.-Phys.
(86) Internationale Anmeldenummer: EP9301794
(87) Internationale Veröffentlichungsnummer: WO9401060

(56) Entgegenhaltungen:
- EP-A- 0 363 753
- WO-A-89/00840
- US-A- 3 714 671
- US-A- 3 755 823

## Beschreibung

Die Erfindung betrifft ein Stent (Stützgehäuse) für implantierbare Klappen- und Schließorgane, insbesondere für Herzklappenprothesen, mit einem Basisring, der mindestens zwei vorzugsweise symmetrisch zueinander versetzte, im wesentlichen in Ringachsrichtung weisende, über bogenförmige, der Befestigung mindestens zweier flexibler Segel dienende Leisten miteinander verbundene Pfosten trägt.

Eines der Hauptprobleme bei der Schaffung einer Prothese aus einem derartigen Stent besteht darin, eine geeignete Konstruktion zu schaffen, bei der die Stentpfosten aus ihrer Herstellungsform oder einer andersartig eingestellten Ausgangsform, gegebenenfalls konisch, im implantierten Zustand durch sehr geringe Kräfte bzw. Schließdruckdifferenzen des Blutstroms in einen zylindrischen Zustand verformt werden können und anschließend eine radial nach innen gerichtete Verformung der Stentpfosten nur mit hohem Kraftaufwand erfolgen kann.

Zum besseren Verständnis wird zunächst das Schließverhalten und die Öffnungsbewegung der natürlichen Aortenklappe beschrieben. Bei der natürlichen Aortenklappe sind die Segel in einem schlauchförmigen Gebilde, der Aortenwurzel integriert. Angrenzend an die Segel befinden sich Aushöhlungen in der Aortenwand, die Aortenbulben. Im Falle einer Prothese wird in der Literatur dies als "Unstented"-Klappe oder Conduitklappenimplantat (häufig ohne Bulben) bezeichnet, im Gegensatz zu den hier beschriebenen Stent-Klappen. Bei Schließverhalten, d.h. in geschlossener Segelstellung kommt diesen Aortenbulben eine festigkeitsmechanische Funktion zu. Ohne die Ausbuchtungen der Bulben wäre die Aortenwand nicht in der Lage, radikale Kraftkomponenten aufzunehmen, um die Kräfte auszugleichen, die auf sie durch die Segel unter diastolischem Druck ausgeübt werden. Diese Kraftkomponenten werden bei künstlichen Stent-Herzklappen durch deren Stentpfosten aufgenommen, die dadurch radial zur Klappenmitte hin verformt werden.

Aus dem unterschiedlichen Aufbau der Stented- und Unstented-Klappe resultiert ein abweichendes Bewegungs- bzw. Öffnungsverhalten der segelförmigen Schließelemente.

In der Unstented-Klappe mit Bulben sind die Kommissuren (im Falle einer Stent-Klappe die Pfostenspitzen) der Segel fest mit der Aortenwurzel verbunden. Aufgrund der Expansionsfähigkeit der Aortenwurzel kann die natürliche Klappe frühzeitig öffnen. Zu Beginn der Systole steigt durch die isovolumetrische Kontraktion der Ventrikeldruck an, wodurch sich die Kommissuren radial nach außen bewegen. Diese Kommissuren-Expansion beginnt schon vor der eigentlichen Klappenöffnung. Die Bewegung der Kommissuren verursacht auf die Segel eine tangential wirkende Kraft. Dadurch nimmt die Segelkrümmung ab, die Segel werden allmählich voneinander getrennt und in eine offene Position gezogen. Die kleinste so hervorgerufene Klappenöffnung ist ohne nachweisbaren vorwärtsgerichteten Fluß und in Abwesenheit eines Aortendruckanstieges zu erkennen. Im Verlauf der Systole nimmt bei steigendem Aortendruck die Expansion der Aortenwurzel weiter zu. Die Aortenklappe bildet somit in der Systole ein Rohr mit wachsendem Durchmesser und stellt unter dein Gesichtspunkt der Hydrodynamik einen Diffusor dar. Daneben dient die Expansionsfähigkeit der Bulbenklappe quasi zur Regulierung der Segel-Überlappungsfläche im geschlossenen Zustand und verhindert so Faltungen innerhalb des Segels bei variierenden physiologischen Belastungszuständen, d.h. unterschiedlichen Schließdruckdifferenzen. Stellt man sich die Segel der natürlichen Klappe in geschlossener Position bei verschiedenen Druckdifferenzen vor, so steigt mit wachsender, maximaler Druckdifferenz über der Klappe gleichzeitig der Aortendruck, und dadurch der Aortendurchmesser in Höhe der Kommissuren an. Als Folge heben sich die Klappensegel stromab. Durch diese Vorgänge nimmt die Größe der Segel-Überlappungsfläche ab. Die Bulbenklappe paßt sich offenbar verschiedenen Belastungszuständen durch variierende Segel-Überlappungsflächen an.

### Stand der Technik

Aus dem Stand der Technik, so z.B. aus der DE 38 34 545 A1 und der DE 38 90 591 T1 sind Stents bekannt, die einen zylindrischen Basisring haben, der durch drei auf dem Umfang jeweils um 120° versetzte, sich in axialer Richtung verengende Pfosten verlängert wird. Die durch die Pfosten gebildeten Einbuchtungen, die einer dreizackigen Krone ähneln, dienen zur Aufnahme der Segel, die die eigentliche Schließfunktion der Klappe übernehmen. Die Einbuchtungen sind je nach Prothese geometrisch verschieden, werden aber häufig der geometrischen Form der Befestigungslinie des natürlichen Klappensegels an die Aortenwand nachgebildet. Die unter Schließdruck geschlossenen Segel bewirken eine radial zur Klappenachse nach innen gerichtete Biegung der mehr oder weniger elastischen Stentpfosten. Dabei strebt der zweitgenannte, bekannte Vorschlag durch einen Verbundaufbau von Polymer und Federstahl der Pfosten sowie Ausnehmungen in deren Fußbereich eine möglichst vollständige Flexibilität an.

Die bekannten Stent-Klappenkonstruktionen haben den Nachteil, daß diese Klappen sich variierenden physiologischen Belastungszuständen, d.h. unterschiedlichen Schließdruckdifferenzen, nur unzureichend anpassen können. Durch die Schließdruckdifferenz im geschlossenen Klappenzustand werden radiale Kraftkomponenten über die Segel auf die Stentpfosten ausgeübt, die dadurch radial zur Klappenmitte verformt werden. Mit zunehmender Schließdruckdifferenz senken sich die Segel stromauf, bauchen sich ein und legen sich entlang ihres freien Randes aneinander, bis eine vollständige Segelüberlappung die Klappe abdichtet. Da eine Stentklappe nur für einen Schließdruckzustand konstruktiv günstig ausgelegt werden kann, nehmen bei über diesen Zustand weiter steigender Druckdifferenz die radialen Pfostenbewegungen die Einbauchungen der Segel und die Segel-Überlappungen weiter zu. Auf Höhe des freien Segelrandes wird die Überlappungsfläche überflüssig groß, so daß unerwünschte Faltungen im Segel entstehen. Dieser Effekt wird zudem durch das nachstehend beschriebene, einerseits bewußte flexible Verhalten und andererseits Kriechen der Stentpfosten verstärkt.

Zur Verringerung von hohen Spannungen in den Segelbereichen (Kommissuren), die an die Stentspitzen angrenzen, wird z.B. in DE 35 41 478 A1 und US 5 037 434 vorgeschlagen, die Stentpfosten in deren oberen Teil sehr flexibel zu konstruieren. Sind jedoch, wie beim Stand der Technik übliche, die Stentpfosten bzw. Spitzen flexibel, werden sie bei der Beanspruchung durch die Schließdruckdifferenz an diesen Stellen übermäßig gedehnt, was schließlich zum Kriechen und dadurch zu frühzeitigem Ermüden führt.

In US-PS 3 755 823 werden Stent-Ausbildungen vorgeschlagen, bei denen die zuvor erwähnte Flexibilität der Stentpfosten praktiziert wird.

Schließlich ist aus US-PS 3 714 671 eine Konstruktion bekannt, bei der jeweils zwei Pfostenenden in ganz bestimmter, geometrischer Relationen einhaltender Weise zusammengefügt werden, ohne daß darin das Flexibilitätsproblem angesprochen wird.

Daher ist es bei Stentklappen nötig, die radial nach innen zur Klappenachse gerichtete Verformung der Stentpfosten, insbesondere ihrer Spitzen, in geschlossener Segelstellung zu begrenzen. Ein starrer Stent würde zwar das Absenken der Segel und die Faltungen verringern, aber nicht den steilen zeitlichen Druckgradienten dp/dt beim Klappenschluß dämpfen.

### Darstellung der Erfindung

Der Erfindung liegt daher die Aufgabe zugrunde, ein Stent der eingangs genannten Art zu schaffen, mit dem ein Klappen- oder Schließorgan, insbesondere eine Herzklappenprothese hergestellt werden kann, die auch bei unterschiedlichen Schließdruckdifferenzen, d.h. insbesondere auch bei variierenden physiologischen Belastungszuständen, zuverlässig arbeitet. Dabei geht die Erindung von der Überlegung aus, durch konstruktive Maßnahmen ein ausgeprägtes, progressives Schließdruck-Pfostenverformungsverhalten zu erzeugen, wobei einerseits bei hohen Schließdruckdifferenzen die Verformung der Pfosten bis zu definierten Positionen begrenzt wird.

Diese Aufgabe wird durch das Stent nach Anspruch 1 gelöst.

Die Erfindung sieht eine anschlagbegrenzte Flexibilität der Pfostenbasen vor, gegebenenfalls ersetzt, jedoch vorzugsweise noch ergänzt durch eine anschlagbegrenzte Flexibilität der Leisten, wodurch eine radiale Verschiebung bzw. Verformung der Pfosten möglich wird, die dann durch einen Anschlag begrenzt wird.

Konkrete Ausgestaltungen mit ihren Vorteilen ergeben sich aus den Ansprüchen sowie der nachfolgenden Beschreibung.

Wie grundsätzlich aus dem Stand der Technik (US-A-3 755 823, US-A-3 714 671 oder WO 89/00840) bekannt, können nach einer Weiterbildung der Erfindung die freien Pfostenenden starr ausgebildet sein, insbesondere durch eine Materialsanhäufung an den freien Pfostenenden, die sich vorzugsweise derart gestalten läßt, daß damit ein Pfostenquerschnitt erreicht wird, der zusammen mit den bogenförmigen Leisten im Unterschied zum Stand der Technik eine nach außen gerichete Fläche zur Anbringung der Segel schafft, die nur einfach gekrümmt ist und somit in überraschender Weise diese erste Alternative noch den Vorteil mit sich bringt, ein ebenflächig hergestelltes Segel auf eine einfach gekrümmte Fläche formschlüssig fügen zu können. Durch die relative Starrheit der Stentpfosten im oberen Bereich werden dort hohe Dehnungen vermieden. Mit der Materialanhäufung in Form einer prismatischen Innenauflage an den Pfostenenden wird zudem in einfacher Form erreicht, daß die auf die einfach gekrümmten Pfosten- und Leistenflächen aufgebrachten Segel - das Verbinden der Segel mit dem Stent kann durch Kleben oder Schweißen geschehen - sich dichtend an die Innenauflage anlegen, ohne daß es zusätzlicher Dichtungsmaßnahmen bedarf, die andernfalls wegen der nach außen gerichteten Befestigungsflächen, die durch den trapezförmigen Pfostenquerschnitt bei nach außen gerichteter kleinerer Trapezseite, entstehen, notwendig wären.

Die anschlagbegrenzte Flexibilität der Pfosten sowie der sie verbindenden Leisten, die vorzugsweise einstückig ausgebildet sind, wobei die Pfosten ihrerseits im Basisbereich Ausnehmungen besitzen, wird generell durch meachanische Formgebungsmaßnahmen in Form von quasi Nut- und Feder-Anschlag erreicht, wozu im Basisbereich ein zusätzlicher Ring (Anschlagring) vorgesehen wird, der vorzugsweise eine umlaufende Nut aufweist, in die im Basisbereich der Pfosten an diesen angebrachte, nach außen gerichtete nasenartige Vorsprünge oder radial umlaufende Lippen eingreifen, während die Leisten in ihrem basisnahen Bereich, also mittig jeweils zwischen zwei Pfosten unterseitig eine der Querschnittsform des Anschlagringes angepaßte, vorzugsweise rechtwinklige Ausnehmung haben, die im Ausgangszustand, bei dem also die Pfosten nicht nach innen verformt sind, vielmehr gegebenenfalls allenfalls leicht nach außen geneigt sind, Abstand vom Anschlagring aufweisen, ebenso wie die vorerwähnten Rippen der Pfostenfüße zur Nut im Anschlagring.

Dabei ist die Gestaltung so gewählt, daß sich die Nut im Anschlagring in einer Höhe befindet, die es erlaubt, daß der obere Nutrand außenseitig als Anschlag für die Leisten und innenseitig als Anschlag für die Pfostenrippen dient. Auf diese Weise wird folglich sichergestellt, daß die nach innengerichtete Verformbewegung der Pfosten bei Erreichen der gewünschten Endstellung über die dann innerhalb der Nut an den Anschlagring anstoßenden Rippen gestoppt wird, was dadurch optimal unterstützt wird, daß bei dieser Bewegung die nach außen gerichtete Bewegung der Verbindungsleisten durch zum Zeitpunkt des Anschlages der Rippen im Leistenbereich ebenfalls erfolgendes Aufsitzen bzw. Anschlagen der Leistenausnehmung auf bzw. an dem Anschlagring zu einer Bewegungsbegrenzung auch dieses flexiblen Stentteils führt. Im Rahmen der Erfindung ist es im Falle eines gewünschten abgestuften Flexibilitätsverhaltens auch möglich, die Anschläge zeitlich versetzt erfolgen zu lassen, was z.B. durch unterschiedliche Abstände der einander zugeordneten Anschlagflächen eingestellt werden kann.

Der Herzklappenstent soll im Herstellungszustand eine Diffusorform aufweisen. Bevorzugt ist dabei an eine konische Form mit Kegelwinkeln zwischen 2° und 14° gedacht.

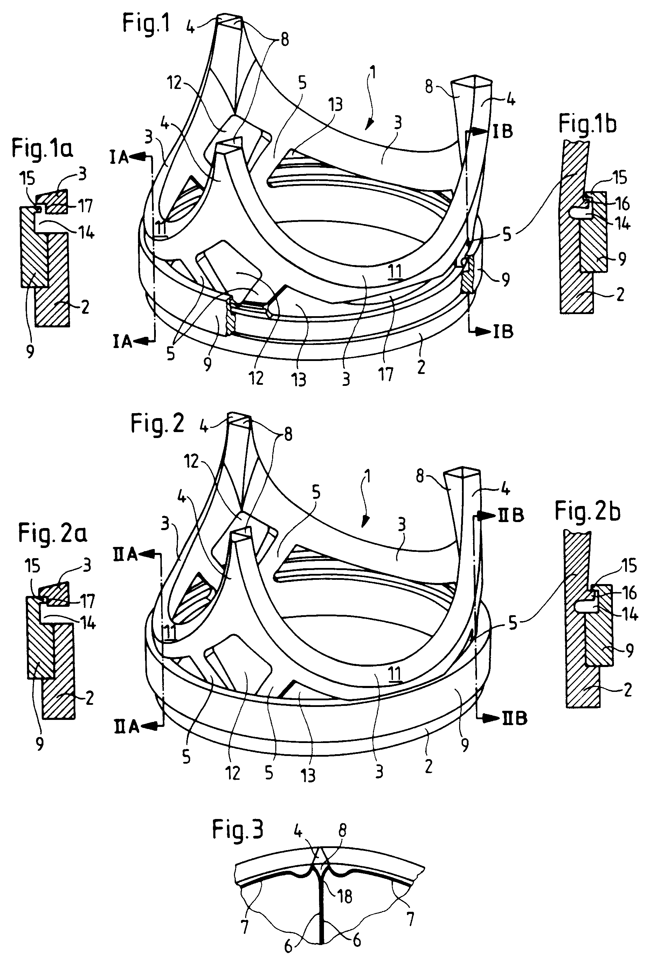

Zur Begrenzung der Stentpfostenkontraktion ist nun erfindungsgemäß vorgesehen, einen zusätzlichen Ring über den Basisring zu plazieren. Der Schließdruck des Blutes übt über die Segel radial zur Klappenmitte gerichtete Kräfte auf die Stentpfosten aus und kontrahiert sie. Da die Segmente im Pfostenbereich relativ starr sind, werden die Schließkräfte vornehmlich in eine Verformung der basisnahen Bereiche der Leisten - im folgenden auch Segmentbögen genannt - umgeleitet. Die Segmentbasisbereiche verformen und verschieben sich gleichzeitig radial nach außen und stromaufseitig, bis eine weitere Verformung durch den eingelegten Ring begrenzt wird, wie dies insbesondere aus den Ausschnittsvergrößerungen der Figuren 1 und 2 hervorgeht. Durch die Kontraktion der Stentpfosten verbiegen sich gleichzeitig auch die V-Stege, insbesondere im dünnwandigen, flexiblen Verbindungsbereich zum Basisring. Insofern handelt es sich technisch um ein Filmgelenk. Der Anschlag ist bevorzugt so ausgelegt, daß die Stentpfosten sich in eine zylindrische Konfiguration verformen. Mit zunehmender Schließdruckdifferenz hängt dann die weitere Kontraktion im wesentlichen von der Querschnittsform und dem Elastizitätsmodul des Stentpfostens ab. Die Stentpfosten sind jedoch relativ starr gewählt. Durch diese konstruktive Auslegung besteht eine Expansions- und mehr oder weniger beschränkte Kontraktionsfähigkeit der Stentpfosten. Die Gitterstruktur zusammen mit der Verformungsbegrenzung durch den Anschlagring verteilt die Belastung des Schließdrucks über die gesamte Länge der Segmente und vermeidet so Spannungsspitzen wie bei bisher üblichen Stents.

Der durch den eingelegten Ring erzeugte "Anschlag" könnte auch direkt im Basisring integriert sein, d.h. Anschlagring und Basisring wären ein einziges Bauteil aus gleichem Werkstoff. Im Falle, daß die Aussparungen der Stentwand mit einem Werkstoff geringeren E-Moduls ausgefüllt sind, würde der Füllwerkstoff zu einer Dämpfung der Kontraktion des Stents führen.

Alternativ oder ergänzend zur Verformungsbegrenzung der Segmentbasisbereiche wird vorzugsweise auch eine direkte Begrenzung der Pfostenbiegung im Bereich der Verbindungsstege vorgesehen. Dazu wird der Steg in Umfangsrichtung mit einem rippenartigen Vorsprung versehen, über den vom Basisring eine Art (Schnapp-)Haken gebracht wird, der die durch den Schließdruck hervorgerufene, zur Stentspitze orientierte Drehung der Vorsprünge bzw. des durch den Steg gebildeten Filmgelenks begrenzt. Die kraftformschlüssige Verbindung beruht also auf dem Ineinanderrasten von Hinterschneidungen, dem Vorsprung auf den Verbindungsstegen einerseits und dem Haken ausgehend vom Basisring andererseits.

Die konstruktive Auslegung der Pfosten und die Werkstoffauswahl ist so bemessen, daß die Grenze der Kontraktionsfähigkeit der Stentpfosten in den zylinderförmigen Zustand vorzugsweise bei Druckdifferenzen von 266 bis 3990 Pa (2 bis 30 mmHg) erreicht wird. Mit darüber hinaus zunehmendem Schließdruck verhält sich der Stent weitgehend starr, d.h. die weitere Kontraktion der Stentkrone wird behindert. Da durch kann sich die Klappe einem größeren Bereich an Schließdruckdifferenzen anpassen und reduziert die zuvor beschriebene unerwünschte weitere Einbauchung und Faltenbildung der Segel.

Nimmt dagegen die Druckdifferenz gegen Null ab, befähigt die Expansion des Stents ein frühzeitiges Öffnen der Segel. Die Pfosten-Expansion beginnt schon vor der eigentlichen Klappenöffnung. Die Bewegung der Pfosten verursacht auf die Segel, insbesondere an den Kommissuren, eine tangential wirkende Kraft. Dadurch nimmt die Segelkrümmung ab, die Segel werden allmählich voneinander getrennt und in eine offene Position gezogen. Die Vergrößerung der Biegeradien der sich öffnenden Segel verringert die Segelspannungen und vermeidet so Spannungskonzentrationen. Überdies bildet der Stent während der Öffnungsphase ein Rohr mit wachsendem Durchmesser und stellt unter dem Gesichtspunkt der Hydrodynamik dafür einen günstigen Diffusor dar.

Ein Konstruktionsdetail des erfindungsgemäßen Stents sind die Flächen der bogenförmigen Einbuchtungen der Krone, d.h. der Leisten, an denen die Segel befestigt werden. Die Verbindungslinie von Segel und Stent, d.h. die bogenförmige Einbuchtung, ergibt sich aus der Durchdringung eines rohrförmigen Stents mit z.B. einer Kugel. Die Verbindungslinien können sich aber auch aus der Durchdringung mit jedem anderen geometrischen Körper ergeben, so daß die beschriebenen bogenförmigen Einbuchtungen entstehen. Diese Flächen sind üblicherweise zweifach gekrümmt. In der hier beschriebenen Art sind die Einbuchtungsflächen im Gegensatz zum Stand der Technik einfach gekrümmt, so daß deren Flächennormalen radial zur Klappenachse zeigen. Die charakteristische Querschnittsform der Stentpfostenspitze ist dadurch trapez- bis dreiecksförmig, wobei die kurze der parallelen Seiten des Trapez, entgegengesetzt zu üblichen Formen, radial nach außen zeigt, wie dies aus den Figuren 1 und 2 hervorgeht. Der Übergang von der einfach gekrümmten Einbuchtungsfläche zur äußeren Zylinderfläche des Stents kann auch leicht gerundet oder mit einer Fase abgeschrägt sein.

Werden die Segel entlang der Durchdringungslinie am Stentinnendurchmesser befestigt, bewirkt diese Trapez-Formgebung insbesondere eine günstige, radiale Strömungsführung um die Stentpfosten. Die Hinterschneidungen, wie sie bei entgegengesetzt gerichteten Trapezformen sonst üblicher Stentpfosten verursacht werden, entfallen.

Die durch die Anordnung der breiten der parallelen Trapezseiten auf dem Innendurchmesser an der Stentspitze sich ergebenden, dreiecksförmigen Rückflußspalte werden durch die erfindungsgemäßen Innenauflagen bzw. Anformungen geschlossen, die sich über die Anbringungsstelle von Segel und Stent in Richtung auf die Klappenmitte erstreckt. In der Draufsicht formt sich die verlängernde Nase dreieckig mit beidseitig konkaver Verjüngung aus. Beginnend von der Anbindungssstelle des Segels an den Stent verjüngt sich der Pfostenquerschnitt also zunächst geradlinig bzw. dreiecksförmig oder konvex und anschließend konkav. Erfolgt die dreiecksförmige Verjüngung mit sehr spitzen Winkeln, kann auf die anschließende konkave Verjüngung verzichtet werden. Nach unten, d.h. in stromaufseitiger Richtung, verjüngt sich die Nase bis zum Beginn der Segmentendverbindungen in die Stentwand.

Die erfindungsgemäße Innenauflage entlastet die Bereiche der Segelkommissuren im geschlossenen Zustand und lenkt sie allmählich mit geringeren Winkeln um, bis sich die Segel direkt aneinanderlegen. So werden hohe Spannungsspitzen vermieden, die bei sonst üblichen Klappenformen in den Segelkommissuren auftreten. Durch die Innenauflage, die sich der Segmentendverbindung anschließen, wird somit zusätzlich zur erfindungsgemäß angestrebten Versteifung der Kronenspitze ein weiterer wesentlicher Vorteil erreicht.

Die Vorteile dieser nasenförmigen Stentspitze kommen auch zum Tragen, wenn die Stentwand keine Aussparungen besitzt, oder die Einbuchtungsflächen von der einfach gekrümmten Form abweichen.

Die vorstehenden Ausführungen gelten auch hinsichtlich der erreichten Vorteile der erfindungsgemäßen Stents in gleicher Weise für eine zwei-, drei- oder mehrseglige Klappe.

Wird eine solche zuvor beschriebene Formgebung des Stents in einen zylindrischen Schlauch integriert oder zusätzlich mit sogenannten Bulben (Sinus valsalvae) ähnlich der natürlichen Aortenklappe gefertigt, kann eine solche Klappenprothese in künstliche Blutpumpen oder als Conduit-Klappenimplantat zum Ersatz geschädigter Aortenklappen eingesetzt werden.

### Kurze Beschreibung der Zeichnung

Nachstehend wird die Erfindung am Beispiel eines Herzklappenstents anhand der Zeichnungen näher erläutert.

Herstellungsmäßig besteht eine Komponente des flexiblen, insgesamt mit 1 bezeichneten Herzklappenstents, wie insbesondere die perspektivischen Darstellungen gemäß den Figuren 1 und 2 zeigen, aus einem zylindrischen Basisring 2 zur Aufnahme eines für das Implantieren erforderlichen, nicht dargestellten, da allgemein bekannten Nahtrings. Eine weitere Herstellungskomponente für den Stent ist eine Art dreizackige, drahtförmige Krone, die aus drei Segmentbögen oder auch Leisten 3 besteht, die an ihren freien Enden in gemeinsame Spitzen der Kronen oder Pfostenenden 4 übergehen, unterhalb derer V-förmig verlaufende Stege 5 von den Segmentbögen bzw. Leisten ausgehen, die mit dem Basisring 2 gegebenenfalls einstückig verbunden sind, so daß ein sehr flexibler Aufbau gegeben ist, da die Verbindung der Pfosten und Leisten bzw. der dreizackigen "Krone" 3, 4 lediglich über diese Stege 5 erfolgt.

Die Befestigung der eigentlichen Schließelemente, nämlich der Segel 6/7 am Stent erfolgt entlang der schräg nach außen weisenden Fläche 11 der Segmentbögen 3 und der Pfostenenden 4. Die V-Stege 5 sind ebenso wie die Pfostenenden 4, die somit zusammen den Pfosten herkömmlicher Bauweise ersetzen, um 120° versetzt angeordnet und stellen somit basisnah durchbrochene Pfosten dar, so daß auch dadurch eine große Flexibilität im Basisbereich gegeben ist, wie sie für die Segmentbögen mangels einer direkten Verbindung mit dem Basisring in diesem Bereich auch gegeben ist; hingegen sind die Leisten bzw. Segmentbögen 3 im Bereich der Pfostenenden 4 konstruktiv relativ starr ausgelegt. Dazu dient auch eine eingangs bereits ausführlich beschriebene Innenauflage 8 im Bereich der Pfostenenden 4, nämlich eine prismatische, sich zur Basis hin verjüngende Innenauflage 8 mit dreieckigem Querschnitt, die mit einer Dreieckseite der Innenfläche der Pfostenenden 4 anliegt. Als Stentpfosten sind somit die Bereiche der V-Stege 5 mit den der Kronenspitze bzw. den Pfostenenden 4 benachbarten Segmentteilen anzusehen.

Da, wie bereits erwähnt, die Basisbereiche der Segmentbögen bzw. Leisten 3 mit dem Basisring 2 nicht verbunden sind, wird größtmögliche Flexibilität, d.h. Kontraktion der Stentpfosten bei sehr geringen Schließdrücken erreicht. Die Aussparungen der gitterförmigen Stentwand werden mit einem Werkstoff ausgefüllt oder überspannt, der gegenüber den sonstigen Stentkomponenten einen wesentlich geringeren Elastizitätsmodul hat. Dies dichtet die Stentwand gegen eine radiale Durchströmung ab. Gegebenenfalls können die Aussparungen auch mit dem eigentlichen Stentwerkstoff geschlossen werden, wenn die Dicke in diesen zu überspannenden Bereichen wesentlich geringer als die der Segmentbögen bzw. Leisten 3 ist. Schließlich gehört zum erfindungsgemäßen Stent noch ein koaxial teilweise um den Basisring 2 gelegter Anschlagring 9, der in zuvor bereits beschriebener und nachfolgend anhand der Zeichnungen noch zu erläuternder Weise für die gewünschte Bewegungsbegrenzung der Stentpfosten sorgt.

Im einzelnen zeigen die beiliegenden Zeichnungen folgendes:

### Wege zur Ausführung der Erfindung

Figur 1 stellt einen erfindungsgemäßen Stent in perspektivischer Ansicht im diffusorförmigen Herstellungszustand, d.h. mit leicht nach außen geneigten Pfosten dar, wobei der koaxial partiell um den Basisring 2 gelegte, und an diesem lagegesicherte Anschlagring 9 im vorderen Bereich teilweise gebrochen dargestellt ist, um seinen Querschnitt mit der räumlichen Zuordnung zu den übrigen Stentteilen, wie sie auch aus den Querschnittdarstellungen gemäß Fig. 1a und 1b aus Fig. 1 hervorgehen, deutlich zu machen. Diese Stentanordnung und -form entspricht der offenen Stellung bei angeformten, nicht dargestellten Segeln, die dann mit der nach außen geneigten, durchgehenden, einfach gekrümmten Fläche der Leisten 3 und Pfostenenden 4 verbunden wären. Die Endverbindungen der Segmentbögen bzw. Leisten 3 bilden die "Kronen"spitzen bzw. Pfostenenden 4.

Die erwähnten Flächen 11 sind einfach gekrümmt, wodurch sich trapezförmige Querschnitte der Pfostenenden ergeben, wobei die kürzere Seite der parallelen Trapezseiten nach außen gerichtet ist, wohingegen an dieser Stelle beim Stand der Technik eine Dreiecksform mit nach innen ragender Spitze vorgesehen ist. Durch die "Kronen"form mit den V-förmig verlaufenden, endseitig mit dem Basisring verbundenen Stege 5 ergibt sich eine gitterförmige Stentwand mit Durchbrechungen 12 und 13, wobei gegebenenfalls auf die Durchbrechung 12 auch verzichtet werden kann. Diese Durchbrechungen 12 und 13 sind für den funktionsfähigen Betrieb der Klappe zu überspannen oder auszufüllen, um unerwünschte Rückflüsse zu vermeiden. Wie erwähnt, gehört zum Stentpfosten hier der Bereich der V-förmig verlaufenden Stege 5 mit den sich zur "Kronen"spitze hin anschließenden Segmentteilen.

Wie den Figuren 1a und 1b bzw. 2a und 2b zu entnehmen ist, weist der Anschlagring 9 eine umlaufende Nut 14 auf, und zwar innenseitig umlaufend in einer derartigen Höhe, daß sie nach oben durch eine Art Rippe 15, die Teil des Anschlagrings ist und ebenfalls vorzugsweise aus Herstellungsgründen umlaufend vorgesehen ist, begrenzt wird. Nicht zuletzt wegen der in der Ausgangsposition gemäß Figur 1 leicht nach außen geneigten Stege 5 (s.insbesondere Fig. 1b) springt die Rippe gegenüber der Innenwandung des Anschlagrings 9 leicht zurück, so daß die Außenneigung der Stege 5 nicht beeinträchtigt wird.

Wie die Figuren 1b und 2b zeigen, sind die V-förmig verlaufenden Stege 5 im Überlappungsbereich mit dem Anschlagring 9 außenseitig mit einem in diesem Bereich vorzugsweise umlaufenden nasenartigen Vorsprung oder radial umlaufenden Lippen 16 versehen, die sich in Höhe der Nut 14 befinden und in diese derart freibeweglich eingreifen, daß sie den oberen Nutrand bzw. die Rippe 15 im Ausgangszustand gemäß Fig. 1b nicht berühren, jedoch im nach innen gebogenen Endzustand der Pfosten gemäß Figur 2b der Rippe 15 unterseitig anliegen, so daß die Rippe zur Begrenzung der Verformung der "Krone" nach innen als Anschlag wirkt.

Ein entsprechender Anschlag ist im bevorzugten Ausführungsbeispiel auch für den basisnahen Bereich der Segmentbögen oder Leisten 3 vorgesehen, wozu auf die Figuren 1a und 2a verwiesen wird. Dazu besitzen die Segmentbögen oder Leisten 3 im basisnahen Bereich jeweils mindestens eine rechtwinklige Ausnehmung 17, die umlaufend so angeordnet sind, daß sie die den Figuren 1a und 2a zu entnehmende Zuordnung gestatten, nämlich den oberen Nutrand bzw. die Rippe 15 im aufgeweiteten Ausgangszustand des Stents nicht berühren, jedoch im Zustand nach Figur 2, dem Klappenschließzustand, dem Nutrand bzw. der Rippe 15 als Anschlag von außen anliegen (s.Figur 2a).

Um den Anschlagring 9 herum würde an diesem der nicht dargestellte Nahtring fixiert, um über letzteren die Klappeneinheit im natürlichen Annulusgewebe einzunähen.

Figur 2 zeigt, wie bereits erwähnt, die erfindungsgemäße Stentausbildung gemäß Figur 1 ebenfalls in perspektivischer Darstellung, jedoch im zylinderförmigen Zustand im Falle angeformter, unter Schließdruck geschlossener Segel, die hier aus Gründen der Übersichtlichkeit nicht eingezeichnet wurden. Durch die Kontraktion der Stentpfosten hat sich der Basisberich der Segmentbögen bzw. Leisten 3 stromaufwärts und radial nach außen verformt und über die Ausnehmung 17 von außen an den im Basisring eingelegten Anschlagring gelegt, während die Lippen 16 der V-Stege 5 innenseitig dem oberen Nutrand bzw. der Rippe 15 anliegen.

Figur 3 zeigt eine ausschnittsweise Draufsicht auf die Stentspitze, in der zudem die an den Anformflächen 11 innerhalb der Segmentbögen 3 befestigten, geschlossenen 6 und geöffneten 7 Segel dargestellt sind. Diese Ansicht verdeutlicht die dreiecksförmige und sich beidseitig zur Klappenmitte konkav-verjüngende Innenauflage 8 der "Kronen"spitze, wodurch eine geringere Umlenkung der geschlossenen Segel erreicht wird. An der Spitze 18 der Innenauflage 8 legen sich die Segel aneinander.

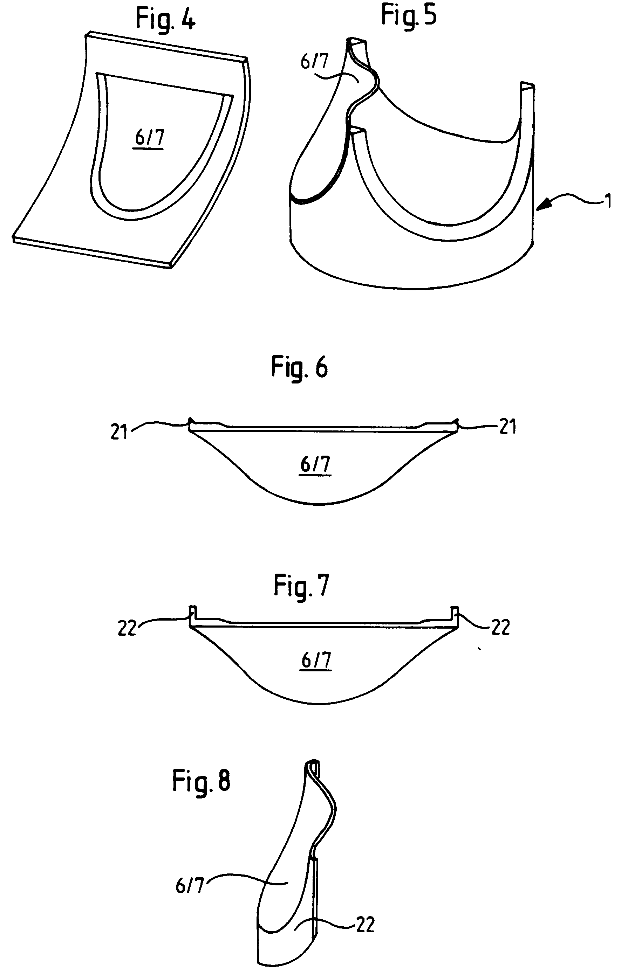

Anhand der Figuren 4 bis 8 werden nachfolgend noch besondere Vorteile des erfindungsgemäßen Stents hinsichtlich der Segelformen und ihrer Anbindung an den Anformflächen 11, die durch einen Füge- bzw. Verbindungsvorgang, sei es Kleben oder Schweißen, erreicht wird, erörtert. Bevorzugt wird ein Verfahren, bei dem ein im wesentlichen ebenes Segel vom Stent getrennt gefertigt und nachträglich an einen nicht aufgespreizten oder an einen im Herstellungszustand gering konischen Stent, der nachträglich nicht durch äußere Kräfte verengt wird, mittels Kleben oder Schrumpfen befestigt wird.

Durch die einfache Krümmung der Anformflächen 11, die durch die erfindungsgemäße, spezielle Trapezform der Stentspitzen erreicht wird, ergibt sich eine gute formschlüssige Verbindung der Segelränder mit den Anformflächen 11.

Die Segel 6/7 (s.Fig. 4) werden im wesentlichen eben ausgeschnitten und gemäß Figur 5 zumindest an den Kontakt- bzw. Berandungsflächen einfach gekrümmt am Stent 1 befestigt. Diese Formgebungen haben dabei den Vorteil, daß die Berandungsflächen der Segel nur sehr wenig verformt werden müssen, um auf den Anformflächen 11 zu liegen. Zudem kann aufgrund der einfachen Krümmung der Anformflächen 11 ein relativ einfaches Halte- und Preßwerkzeug benutzt werden, das die Ränder des Segels an den Stent drückt.

Dieses Verfahren, das eine paßgenaue Plazierung eines so kleinen, flexiblen Bauteils, wie des Segels, erfordert, kann dadurch vereinfacht werden, daß gemäß Figur 6, die eine Draufsicht auf die freie Oberkante des Segels zeigt, auf der zu den Stentflächen orientierten Innenseite entlang des gebogenen Randes des Segels eine z.B. dreiecksförmige Kante 21 verläuft, die in eine entsprechende Fase auf den Anformflächen 11 paßt. Zur Verbindung wird das Segel 6/7 auf den Stent 1 gelegt und zunächst mit geringer Kraft mit einem entsprechenden Werkzeug gegen die Anformflächen 11 des Stents 1 gepreßt. Danach wird ein geringer Schließdruck auf das Segel ausgeübt, das dann quasi entlang seines Randes "einschnappen" kann, so daß dann beispielsweise ein Schweißvorgang die Fügung beenden kann.

Eine weitere Möglichkeit ist in Figur 7 dargestellt, bei der das Segel über die Ränder der Anformflächen 11 hinaus verlängert wird, in Form eines den rundbogenförmigen Rand des Segels begrenzenden, winklig zur Segelfläche zu seiner Konvexfläche hin gerichteten Randes 22. Die auf diese Weise "vergrößerte" Kante vereinfacht den "Schließdruck/Schnapp-Vorgang" des Segels beim Anbringen an den Stent. Figur 8 zeigt ein derartiges Segel im gefügten Zustand in perspektivischer Seitenansicht, woraus deutlich wird, daß der Rand 22 in die Stentmantelfläche übergeht bzw. dieser dicht und paßgerecht anliegt, wodurch sich die erwähnte "Schnapp"-Eigenschaft ergibt.

In Weiterführung können dann drei Segel zusammenhängend z.B. als schlauchförmiges Gebilde gefertigt werden, wodurch die formschlüssige Fügung der Segelfolie an den Stent weiter vereinfacht wird.

## Patentansprüche

1. Stent (1) für implantierbare Klappen- und Schließorgane, insbesondere für Herzklappenprothesen, mit einem Basisring (2), der mindenstens zwei vorzugsweise symmetrisch zueinander versetzte, im wesentlichen in Ringachsrichtung weisende, über bogenförmige, der Befestigung mindestens zweier flexibler Segel dienende Leisten (3) miteinander verbundene Pfosten (4) trägt, gekennzeichnet durch eine anschlagbegrenzte Flexibilität der Pfostenbasen und/oder der Leisten (3).

2. Stent nach Anspruch 1, gekennzeichnet durch eine starre Ausbildung der freien Pfostenenden, vorzugsweise durch eine Materialanhäufung.

3. Stent nach Anspruch 1 oder 2, gekennzeichnet durch eine prismatische Innenauflage an den freien Pfostenenden.

4. Stent nach Anspruch 3, dadurch gekennzeichnet, daß die im Querschnitt dreieckige Innenauflage (8) in Form eines zur Stentbasis spitz zulaufenden Prismas sich beidseitig konkav verjüngt.

5. Stent nach Anspruch 3 oder 4, dadurch gekennzeichnet, daß bei trapezförmigem Pfostenquerschnitt mit außenliegender kleinerer Trapezseite die Innenauflage (8) der größeren Trapezseite derart anliegt, daß sie mit der Dreieckspitze ihres Querschnittes nach innen weist.

6. Stent nach einem oder mehreren der Ansprüche 3 bis 5, dadurch gekennzeichnet, daß sich die Innenauflage (8) im Querschnitt von den Segelanbindungsstellen aus zunächst dreiecksförmig oder beidseitig-konvex und anschließend weiter dreiecksförmig oder beidseitig-konkav verjüngt, so daß die Kommissurenbereiche der geschlossenen Segel allmählich mit geringeren Biegewinkeln umgelenkt und zudem durch die Innenauflageflügel unterstützt werden, die sich stromaufseitig in die Stentwand verjüngen.

7. Stent nach einem oder mehreren der Ansprüche 3 bis 5, gekennzeichnet durch eine einstückige Ausbildung der Pfosten (4/5) mit ihren jeweiligen Innenauflagen (8).

8. Stent nach einem oder mehreren der Ansprüche 1 bis 7, dadurch gekennzeichnet, daß Pfosten (4/5) und Leisten (3) an den Übergängen zwischen den Segelbefestigungsflächen (Anformflächen) (11) und der zylindrischen Stent-Außenwand abgerundet sind.

9. Stent nach einem oder mehreren der Ansprüche 1 bis 7, dadurch gekennzeichnet, daß die Anformflächen (11) der Leisten (3) und der pfostenenden (4) für die Segel (6/7) zwischen der Innen- und der Außenwand des Stent (1) einfach gekrümmt, nach außen geneigt und dabei tangential zum Stent (1) orientiert sind.

10. Stent nach einem oder mehreren der Ansprüche 1 bis 9, dadurch gekennzeichnet, daß die Pfosten (4/5) in ihrem Basisbereich derart ausgenommen sind, daß sie und die ihre Spitzen (4) zu einer Art Krone verbindenden, zum Basisring (2) hin bogenförmig verlaufenden Leisten (3) nur über Stege (5) mit dem Basisring (2) verbunden sind.

11. Stent nach Anspruch 10, gekennzeichnet durch je einen vertikalen Steg unterhalb jedes Pfostenendes (4).

12. Stent nach Anspruch 10, gekennzeichnet durch V-förmig verlaufende Stege (5) unterhalb jedes Pfostenendes (4), und zwar jeweils ein Pfosten (4/5) über zwei Stege (5) für eine größtmögliche Flexibilität (radiale Kontraktionsfähigkeit der Stentpfosten) bei minimalen Druckdifferenzen.

13. Stent nach einem oder mehreren der Ansprüche 1 bis 12, gekennzeichnet durch einen koaxialen Anschlagring (9) im Bereich des Basisrings (2).

14. Stent nach Anspruch 12, dadurch gekennzeichnet, daß der Anschlagring (9) und der Basisring (2) einstückig miteinander verbunden sind.

15. Stent nach Anspruch 13 oder 14, gekennzeichnet durch eine umlaufende Nut im Anschlagring (9).

16. Stent nach Anspruch 15, gekennzeichnet durch mindestens eine in die Nut (14) des Anschlagrings (9) freibeweglich eingreifende, zumindest partiell umlaufende, außenseitige Lippe (16) im Basisbereich des Stentpfostens.

17. Stent nach Anspruch 16, dadurch gekennzeichnet, daß die Lippe bzw. Lippen (16) außenseitig an den Stegen (5) vorgesehen sind.

18. Stent nach einem oder mehreren der Ansprüche 15 bis 17, gekennzeichnet durch mindestens eine, zumindest partiell umlaufende Ausnehmung (17) an den Segmentbögen bzw. Leisten (3), und/oder am Anschlagring (9) mit einer derartigen Zuordnung, daß der Ring (9) als Anschlag für die Leisten (3) dient.

19. Stent nach einem oder mehreren der Ansprüche 1 bis 18, gekennzeichnet durch eine Diffusorform.

20. Stent nach Anspruch 19, gekennzeichnet durch eine zu den Pfostenenden (4) hin sich öffnende konische Form mit Kegelwinkeln zwischen 2° und 14° bei einer Begrenzung der Kontraktion des Stents in eine Zylinderkonfiguration.

21. Stent nach einem oder mehreren der Ansprüche 1 bis 20, dadurch gekennzeichnet, daß die Grenze der Kontraktionsfähigkeit der Stentpfosten (3/4) in den zylinderförmigen Zustand bei Druckdifferenzen zwischen 266 und 3990 Pa (2 und 30 mm Hg) erfolgt.

22. Stent nach einem oder mehreren der Ansprüche 1 bis 21, dadurch gekennzeichnet, daß er in ein Conduit-Klappenimplantat integriert ist, das als stromauf- und stromabseitige Verlängerung des Stents die Form eines zylindrischen Schlauches annimmt.

## Claims

1. Stent (1) for implantable valve and closure members, in particular for heart valve protheses, having a base ring (2) which carries at least two posts (4), preferably symmetrically offset relative to one another, substantially oriented in the direction of the ring axis and linked together by curved strips (3) for securing at least two flexible webs, characterised by the bases of the posts and/or the strips having a flexibility limited by stops.

2. Stent according to claim 1, characterised by a rigid form of the free ends of the posts, preferably by an accumulation of material.

3. Stent according to claim 1 or 2, characterised by a prismatic inner overlay on the free ends of the posts.

4. Stent according to claim 3, characterised in that the inner overlay (8) has a triangular cross-section and tapers concavely on both sides, in the form of a prism tapering to a point towards the base of the stent.

5. Stent according to claim 3 or claim 4, characterised in that the posts have a trapezium cross-section with the shorter side of the trapeziform on the outside and that the inner overlay (8) adjoins the longer side of the trapezium, with the triangular apex of its cross-section pointing inwards.

6. Stent according to one or more of claims 3 to 5, characterised in that the inner overlay (8) tapers in cross-section from the web attachement positions at first triangularly or convexly on both sides and then further triangularly or concavely on both sides, so that the commissure region of the closed web gradually deviates with small bending angles and is in addition supported by the wings of the inner overlay, which taper into the stent wall at the upstream end.

7. Stent according to one or more of claims 3 to 5, characterised by the posts (4/5) being formed integrally with their respective inner overlays (8).

8. Stent according to one or more of claims 1 to 7, characterised in that posts (4/5) and strips (3) are rounded off at the transition between the web securing surfaces (backing surfaces) (11) and the cylindrical outer wall of the stent.

9. Stent according to one or more of claims 1 to 7, characterised in that the backing surfaces (11) of the strips (3) and of the ends of the posts (4) for receiving the webs (6/7) are singly curved between the inner and outer walls of the stent (1), are inclined outwardly and are oriented tangentially to the stent (1)

10. Stent according to one or more of claims 1 to 9, characterised in that the posts (4/5) have apertures in their base region such that they and the strips (3) which connect their tips to a kind of crown and are arched towards the base ring (2) are only connected to the base ring (2) by the way of stays (5).

11. Stent according to claim 10, characterised by having a respective vertical stay under each post end (4).

12. Stent according to claim 10, characterised by having stays (5) in the form of a V under each post end (4), each post (4/5) being connected by way of two respective stays (5) for the greatest possible flexibility (radial contractibility of the stent posts) with minimal pressure differentials.

13. Stent according to one or more of claims 1 to 12, characterised by having a coaxial stop ring (9) in the region of the base ring (2).

14. Stent according to claim 12, characterised in that the stop ring (9) and the base ring (2) are connected integrally together.

15. Stent according to claim 13 or claim 14, characterised by having a circumferential groove in the stop ring (9).

16. Stent according to claim 15, characterised by having at least one at least partially circumferential lip (16) on the outside in the base region of the stent post engaging freely movably in the groove (14) in the stop ring (9).

17. Stent according to claim 16, characterised in that the lip or lips (16) is or are provided on the outer side of the stays (5).

18. Stent according to one or more of claims 15 to 17, characterised by at least one at least partially circumferential recess (17) in the segment bows or strips (3), and/or in the stop ring (9) associates in such a way that the ring (9) serves as stop for the strips (3).

19. Stent according to one or more claims 1 to 18, characterised by having a diffuser shape.

20. Stent according to claim 19, characterised by having a conical shape opening towards the post ends (4) with cone angles between 2° and 14°, the contraction of the stent into a cylindrical configuration being limited.

21. Stent according to one or more of claims 1 to 20, characterised in that the limit of the contractibility of the stent posts (3/4) into the cylindrical state is reached at pressure differentials between 266 and 3990 Pa (2 and 33 mm Hg).

22. Stent according to one or more of claims 1 to 21, characterised in that it is integrated in a conduit valve implant which takes the form of a cylindrical hose forming a prolongation of the stent on the upstream and downstream sides.

## Revendications

1. Stent (1) pour des organes valvulaires et obturateurs qui peuvent être implantés, en particulier pour des prothèses valvulaires cardiaques, comprenant un anneau de base (2) qui porte du moins deux poteaux (4), de préférence décalés symétriquement l'un par rapport à l'autre, montrant pour l'essentiel dans la direction de l'axe de l'anneau et reliés entre eux par l'intermédiaire de listels (3) en arc qui servent à la fixation de deux voiles flexibles du moins, caractérisé par le fait que les bases des poteaux et/ou des listels (3) ont une flexibilité limitée par butée.

2. Stent selon la revendication 1, caractérisé par une formation rigide des extrémités libres des poteaux, de préférence par une accumulation de matière.

3. Stent selon la revendication 1 ou 2, caractérisé par un appui intérieur prismatique aux extrémités libres des poteaux.

4. Stent selon la revendication 3, caractérisé par le fait que l'appui intérieur (8) qui. en coupe transversale, est triangulaire se rétrécit de manière concave des deux côtés, sous forme d'un prisme s'effilant vers la base du stent.

5. Stent selon la revendication 3 ou 4, caractérisé par le fait que les poteaux présentent une section trapézoïdale avec le côté plus petit du trapèze, situé à l'extérieur et que l'appui intérieur (8) s'appuie sur le côté plus grand du trapèze de telle manière qu'il montre vers l'intérieur avec la pointe triangulaire de sa section.

6. Stent selon l'une ou plusieurs des revendications 3 à 5, caractérisé par le fait que l'appui intérieur (8) se rétrécit, en coupe transversale, à partir des points d'attache des voiles, d'abord de façon triangulaire ou convexe des deux côtés et continue ensuite à se rétrécir de manière triangulaire ou concave des deux côtés, de sorte que les zones de commissure des voiles fermées sont déviées petit à petit avec des angles de pliage plus faibles et sont de plus supportées par les ailes de l'appui intérieur, qui se rétrécissent en amont dans la paroi du stent.

7. Stent selon l'une ou plusieurs des revendications 3 à 5, caractérisé par le fait que les poteaux (4/5) sont réalisés en une seule pièce avec leurs appuis intérieurs respectifs (8).

8. Stent selon l'une ou plusieurs des revendications 1 à 7, caractérisé par le fait que les poteaux (4/5) et les listels (3) sont arrondis aux transitions entre les surfaces de fixation des voiles (surfaces d'appui (11) et la paroi extérieure cylindrique du stent.

9. Stent selon l'une ou plusieurs des revendications 1 à 7, caractérisé par le fait les surfaces d'appui (11) des listes (3) et des extrémités des poteaux (4), destinées à recevoir les voiles (6/7) sont courbées de façon simple entre les parois intérieure et extérieure du stent (1), sont inclinées vers l'extérieur et sont orientées tangentiellement vers le stent (1).

10. Stent selon l'une ou plusieurs des revendications 1 à 9, caractérisé par le fait que les poteaux (4/5) sont évidés dans leur zone de base de telle manière que ceux-ci ainsi que les listels (3) qui relient leurs pointes (4) de manière à former une sorte de couronne et s'étendent en arc vers l'anneau de base (2), ne sont reliés avec l'anneau de base (2) que par l'intermédiaire d'entretoises (5).

11. Stent selon la revendication 10, caractérisé par respectivement une entretoise verticale au-dessous de chaque extrémité de poteau (4).

12. Stent selon la revendication 10, caractérisé par des entretoises (5) s'étendant en V au-dessous de chaque extrémité de poteau (4), à savoir respectivement un poteau (4/5) par le biais de deux entretoises (5) pour une flexibilité aussi grande que possible (capacité de contraction radiale des poteaux du stent) à des différences minimales de pression.

13. Stent selon l'une ou plusieurs des revendications 1 à 12, caractérisé par un anneau coaxial de butée (9) dans la zone de l'anneau de base (2)

14. Stent selon la revendication 12, caractérisé par le fait l'anneau de butée (9) et l'anneau de base (2) sont reliés entre eux en une seule pièce.

15. Stent selon la revendication 13 ou 14, caractérisé par une rainure circonférentielle dans l'anneau de butée (9).

16. Stent selon la revendication 15, caractérisé par du moins une lèvre (16) au moins en partie circonférentielle située sur la face extérieure, dans la zone de base du poteau du stent, qui engrène à mouvement libre dans la rainure (14) de l'anneau de butée (9).

17. Stent selon la revendication 16, caractérisé par le fait que la lèvre ou bien les lèvres (16) et ou bien sont prévue(s) sur la face extérieure des entretoises (5).

18. Stent selon l'une ou plusieurs des revendications 15 à 17, caractérisé par du moins un évidement (17) au moins en partie circonférentiel, situé sur les segments en arc ou bien listels (3), et/ou sur l'anneau de butée (9) avec une telle association que l'anneau (9) sert de butée aux listels (3).

19. Stent selon l'une ou plusieurs des revendications 1 à 18, caractérisé par une forme de diffuseur.

20. Stent selon la revendication 19, caractérisé par une forme conique s'ouvrant vers les extrémités des poteaux (4), avec des angles de cône compris entre 2° et 14°, la contraction du stent dans une configuration cylindrique étant limitée.

21. Stent selon l'une ou plusieurs des revendications 1 à 20, caractérisé par le fait que la limite de la capacité de contraction des poteaux (3/4) du stent dans l'état cylindrique est atteinte à des différences de pression comprises entre 266 et 3990 Pa (2 et 30 mm Hg).

22. Stent selon l'une ou plusieurs des revendications 1 à 21, caractérisé par le fait qu'il est intégré dans un implant valvulaire conduit qui, en tant que prolongation amont et aval du stent, prend la forme d'un tuyau cylindrique.
